# EUROPEAN PATENT APPLICATION

(11) **EP 4 625 240 A1**
(43) Date of publication of application: **01.10.2025**
(21) Application number: 25164853.1
(22) Date of filing: 19.03.2025
(51) Int. Cl.: G06F 40/166, G06F 40/30, G16H 10/60, G16H 15/00, G06F 40/56

(54) **TEXT GENERATION SYSTEM, TEXT GENERATION METHOD, AND STORAGE MEDIUM**

(30) Priority: 27.03.2024 JP 2024050946
(71) Applicant: CANON KABUSHIKI KAISHA, Tokyo 146-8501 (JP)
(72) Inventor: UEDA, Ryuta, Tokyo, 146-8501 (JP); SOEDA, Gakuya, Tokyo, 146-8501 (JP)
(74) Representative: Canon Europe Limited

(57) **Abstract**

A text generation system according to the present invention includes an input candidate information acquisition unit configured to acquire input candidate information to be input to a language model configured to output text information from input information, a text information acquisition unit configured to acquire a plurality of pieces of text information different from one another by inputting input information based on the input candidate information to the language model, and a determination unit configured to determine a text candidate by using the plurality of pieces of text information.

## Description

### BACKGROUND OF THE INVENTION

### Field of the Invention

The present invention relates to text generation systems, text generation methods, and storage mediums.

### Description of the Related Art

In the medical field, the creation of electronic medical records has been carried out by doctors and technicians, and labor-saving measures such as voice input have been promoted. However, writing the text still needed to be performed manually.

To further reduce the burden on healthcare professionals, the application of deep learning technology is being considered. It is expected that this technology will be applied not only to the interpretation of medical images but also to the writing of electronic medical records in the future.

### SUMMARY OF THE INVENTION

In the creation of electronic medical records, a technique for generating text is required. For example, it is conceivable to use deep learning technology represented by generative AI discussed in OpenAI's "GPT-4 Technical Report", arXiv:2303.08774v3, 2023. However, in text generation based on deep learning, inference results can vary probabilistically (randomly) and can be significantly affected by minor differences in input information, leading to instability, which is an issue.

The present invention has been made in view of the above and is directed to providing a text generation system, a text generation method, and a storage medium that control the instability of inference results when deep learning is applied to text generation to generate text candidates for stable input into reports and the like.

In addition, realization of a beneficial effect derived from the constituent elements described in the below embodiments of the present invention, which cannot be acquired from a conventional technique, can also be positioned as another purpose of disclosure of the present specification.

According to a first aspect of the present invention, there is provided a text generation system as specified in claims 1 to 13. According to a third aspect of the present invention, there is provided a text generation method as specified in claim 14. According to a fourth aspect of the present invention, there is provided a storage medium as specified in claim 15.

Further features of the present invention will become apparent from the following description of embodiments with reference to the attached drawings.

### BRIEF DESCRIPTION OF THE DRAWINGS

Fig. 1 is a block diagram illustrating a functional configuration of a text generation system according to a first embodiment.
Fig. 2 is a flowchart illustrating an example of processing procedures to be executed by the text generation system according to the first embodiment.
Fig. 3 is a diagram illustrating examples of input candidate information according to the first embodiment.
Fig. 4 is a diagram illustrating examples of text information according to the first embodiment.
Fig. 5 is a diagram illustrating an example of a medical record to which a text candidate according to the first embodiment is input.
Fig. 6 is a diagram illustrating an example of a medical record to which a text candidate according to a variation example 1-1 is input.
Figs. 7A to 7C are diagrams illustrating examples of a medical record to which a text candidate according to the variation example 1-1 is input.
Fig. 8 is a block diagram illustrating a functional configuration of a text generation system according to a second embodiment.
Fig. 9 is a flowchart illustrating an example of processing procedures to be executed by the text generation system according to the second embodiment.
Figs. 10A and 10B are diagrams illustrating examples of an input dialogue for input candidate information according to the second embodiment.
Figs. 11A and 11B are diagrams illustrating examples of input information according to the second embodiment.
Figs. 12A and 12B are diagrams illustrating examples of text information according to the second embodiment.
Fig. 13 is a diagram illustrating an example of a medical record to which a text candidate according to the second embodiment is input.
Figs. 14A to 14C are diagrams illustrating examples of a medical record to which a text candidate according to a variation example 2-1 is input.

### DESCRIPTION OF THE EMBODIMENTS

Hereinafter, embodiments of the present invention are illustratively described in detail with reference to the accompanying drawings. Each of the embodiments of the present invention described below can be implemented solely or as a combination of a plurality of the embodiments or features thereof where necessary or where the combination of embodiments or features from individual embodiments in a single embodiment is beneficial. Constituent elements described in the embodiments are merely examples. Thus, the technical scope of the present invention is determined by the scope of the claims, not limited by the following individual embodiments.

A first embodiment of the present invention will be described. A text generation system according to the first embodiment generates text candidates from text input by a user. The text candidates may be text candidates for report text to be described in medical records (hereinafter, such text candidates are referred to as "report text candidate", "candidate for report text (sentences)", etc.), from text input by a user, which is an example of a text candidate. The present system has a function of performing inference using a language model based on sentences input by the user, and obtaining text information that serves as a report text candidate. It is assumed that the language model used here outputs results that are inconsistent even if the same sentence is input. This system performs inference for a plurality of times on the same input sentence using a language model to obtain a plurality of pieces of different text information. The present system integrates the plurality of pieces of different text information obtained from the language model, thus creating more stable report text candidates, which is a characterizing feature of the present system.

According to the present invention, as compared with creating report text candidates simply by inputting the user's input into the language model, report text candidates with increased accuracy are provided to the user.

In the present embodiment, a description will be provided of an example where candidates for report sentences to be included in a diagnosis section is generated from medical history and/or findings report input by the user as a radiology report.

Even reports where medical history and/or findings is/are not clearly defined, sentences other than diagnostic imaging reports, or non-text information such as medical images referenced during radiographic interpretation, can still achieve the effects of the present invention if they can be used for inference by the language model.

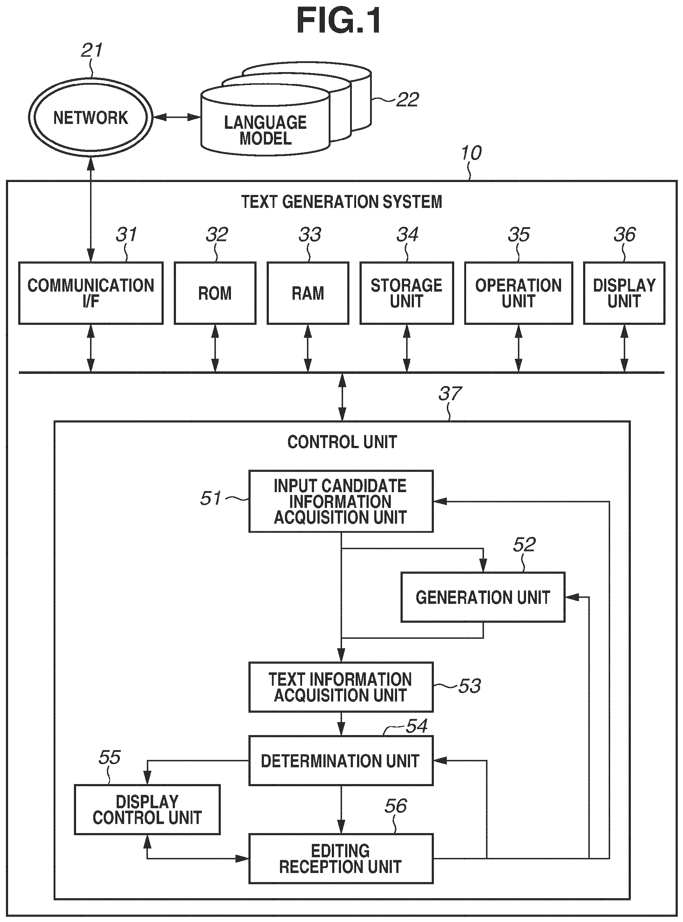

Hereinafter, a functional configuration of a text generation system 10, including the text generation system according to the present embodiment, and processes which are executed by the text generation system 10 are described with reference to Fig. 1. Fig. 1 is a block diagram illustrating an example of the configuration of the text generation system 10 according to the present embodiment. The text generation system 10 is communicably connected to at least one language model 22 via a network 21.

The network 21 includes, for example, a local area network (LAN) and a wide area network (WAN).

The language model 22 has a function of generating text from structured text, keywords, unstructured text, and/or images. The language model 22 has a function of predicting appropriate text as responses based on randomness and probability from the input information. For example, the language model 22 is implemented by a generative pre-trained transformer (GPT) or a bidirectional encoder representations from transformers (BERT). The text generation system 10 can acquire text predicted by the language model 22 via the network 21.

The text generation system 10 includes a communication interface (I/F) 31 (communication unit), a read only memory (ROM) 32, a random access memory (RAM) 33, a storage unit 34, an operation unit 35, a display unit 36, and a control unit 37.

The communication I/F 31 (communication unit) includes a LAN card, and implements communication between the text generation system 10 and an external apparatus, such as the language model 22. The ROM 32 includes a non-volatile memory, and stores various programs. The RAM 33 includes a volatile memory, and temporarily stores various types of information as data. The storage unit 34 includes a hard disk drive (HDD), and stores various type of information as data. The operation unit 35 includes a keyboard, a mouse, and a touch panel, and inputs instructions from the users (e.g., a doctor and a radiogram interpretation doctor) to various apparatuses.

The display unit 36 includes a display, and displays various types of information to the user. The control unit 37 includes a central processing unit (CPU), and generally controls the processing executed by the text generation system 10. The control unit 37 includes an input candidate information acquisition unit 51, a generation unit 52, a text information acquisition unit 53, a determination unit 54, a display control unit 55, and an editing reception unit 56 as functional constituent elements.

The input candidate information acquisition unit 51 acquires information (input candidate information) as a processing target from the operation unit 35. For example, a report created by a doctor or a radiogram interpretation doctor corresponds to the information as a processing target. The input candidate information acquisition unit 51 corresponds to an example of an input candidate acquisition unit configured to acquire input candidate information. In the present embodiment, a radiology report to which medical history and/or findings have been input is acquired as the input candidate information. Alternatively, a radiology report to which information other than the medical history and/or findings has been input may also be acquired as the input candidate information. Further, text other than the radiology report may also be acquired as the input candidate information. The input candidate information is not limited to text information, and may include medical information other than text, such as medical images to be referred during radiographic interpretation.

The generation unit 52 generates input information to be used to cause the language model 22 to execute inference from the text acquired by the input candidate information acquisition unit 51. The generation unit 52 corresponds to an example of a generation unit configured to generate input information to be input to the language model from the input candidate information.

The text information acquisition unit 53 inputs the input information generated by the generation unit 52 to the language model 22 a plurality of times, and acquires results of inferences performed by the language model 22 based on each piece of the input information. It is assumed that the language model 22 according to the present embodiment has randomness, and the output sentences may vary even with the same input. In other words, a plurality of pieces of text information acquired by the text information acquisition unit 53 are not always the same, and can be different from one another. In other words, the text information acquisition unit 53 corresponds to an example of a text information acquisition unit configured to acquire a plurality of pieces of text information different from one another from the language model 22 based on input information.

The determination unit 54 generates candidates for report text to be included in a medical record from the plurality of pieces of text information acquired by the text information acquisition unit 53 by using information frequently included in each of the pieces of text information. For example, the determination unit 54 generates each of the report text candidates by integrating the pieces of text information. The determination unit 54 may internally perform the integration, or an external function such as the language model 22 may perform the integration. The determination unit 54 corresponds to an example of an integration processing unit configured to integrate the pieces of text information acquired by the text information acquisition unit 53.

The display control unit 55 displays report text candidate(s) determined by the determination unit 54 on the display unit 36. Further, the display control unit 55 changes the displayed information according to the operation performed by the user on the below-described editing reception unit 56.

The editing reception unit 56 receives information about editing performed by the user on a report text candidate displayed on the display unit 36 by the display control unit 55. The report text candidates may directly be displayed on the diagnosis section of the radiology report, or may be displayed on another area and reflected on the diagnosis section of the radiology report based on the editing operation performed by the user. In other words, the editing reception unit 56 corresponds to an example of an editing reception unit configured to reflect, into a radiology report, the result of the user editing the report text candidate generated by the determination unit 54. The expression "editing" here refers to, for example, instructions to confirm corrections, such as addition and/or deletion of sentences, to report text candidates, and to confirming reflection of a report text candidate on a radiology report.

The above-described constituent elements included in the text generation system 10 function in accordance with computer programs. For example, the control unit 37 (CPU) reads and executes a computer program stored in the ROM 32 or the storage unit 34 using the RAM 33 as a work area, thus implementing functions of the respective constituent elements. All or part of the functions of the constituent elements in the text generation system 10 may be implemented by a dedicated circuit. Part of functions of constituent elements included in the control unit 37 may be implemented by a cloud computer.

For example, an arithmetic apparatus located in a place different from the place the text generation system 10 is located may communicatively be connected to the text generation system 10 via the network 21. The functions of the respective constituent elements included in the text generation system 10 or the control unit 37 may be implemented through communication between the text generation system 10 and the arithmetic apparatus.

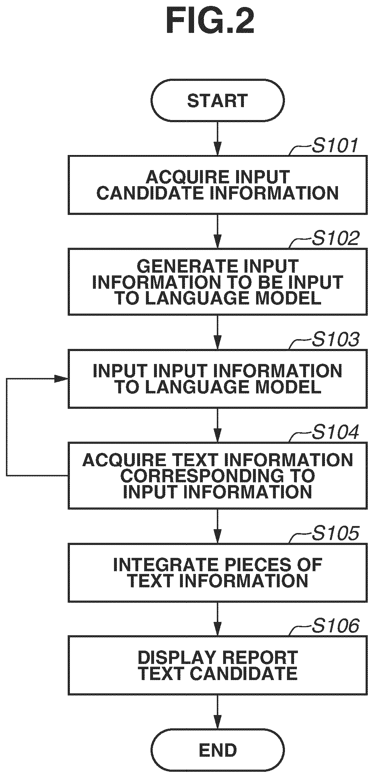

Next, an example of a process of generating report text candidates to be executed by the text generation system 10 according to the present embodiment is described with reference to Fig. 2.

Fig. 2 is a flowchart illustrating an example of processing procedures executed by the text generation system 10. In the present embodiment, a description will be provided of an example in which candidates for report sentences to be described in a diagnosis section of a radiology report are generated from the information about medical history and/or findings described in the radiology report. The present embodiment is also applicable to a case where inference is performed based on text for which distinction between medical history and/or findings is not clear, text other than radiology reports, or non-text information, such as medical images to be referred during radiographic interpretation.

### (Step S101: Acquire Input Candidate Information)

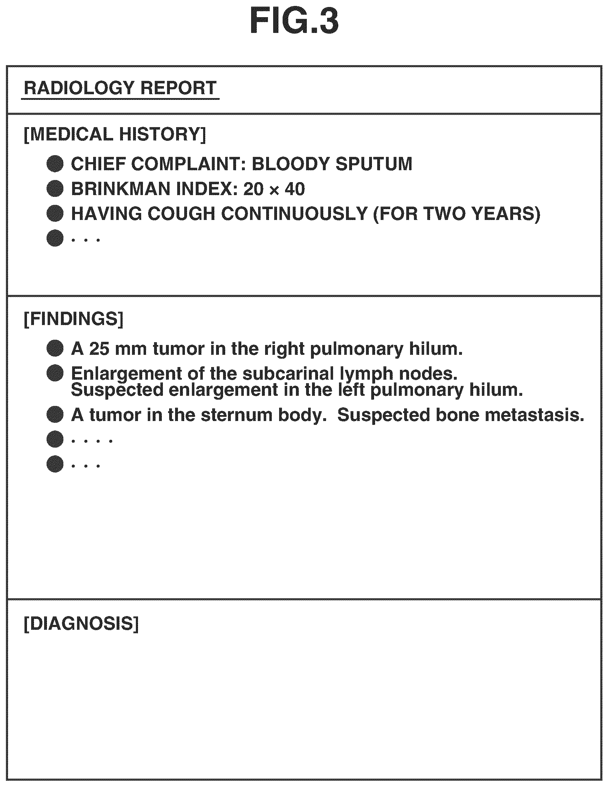

In step S101, the input candidate information acquisition unit 51 acquires the text of medical history and/or findings of a radiology report input by the user via the operation unit 35, and stores the acquired text in the RAM 33. An example of the radiology report input by the user is illustrated in Fig. 3. In the example of the radiology report illustrated in Fig. 3, reports written by a radiogram interpretation doctor as a user are displayed in the medical history column and the findings column, and no report is displayed in the diagnosis section. In the present embodiment, a radiology report as illustrated in Fig. 3 is acquired as the input candidate information.

### (Step S102: Generate input information)

In step S102, the generation unit 52 processes input candidate information acquired by the input candidate information acquisition unit 51 into input information that can be inferred by the language model 22. The operation in step S102 is equivalent to pre-processing of text information acquisition processing to be performed in the subsequent stage, and aims to set details of inference instruction to the input candidate information.

In the present embodiment, the generation unit 52 generates text by adding details of the inference instruction "Generate text for {diagnosis} from the following {medical history} and {findings}. Text in {Diagnosis} should be in bullet points." to the beginning of the input candidate information illustrated in Fig. 3, and this is used as the input information for the language model 22 (hereinafter simply referred to as input information). The information added to the input candidate information is not limited to the above string, as long as it can convey the inference instruction to the language model 22. Alternatively, information added to the input candidate information may be in a non-string form such as parameter information.

### (Step S103: Enter input information to language model)

In step S103, the text information acquisition unit 53 inputs the input information generated by the generation unit 52 to the language model 22 via the communication I/F 31 and the network 21. Thus, inference is executed by the language model 22.

### (Step S104: Acquire text information from language model)

In step S104, the text information acquisition unit 53 acquires an inference result acquired by the language model 22 in step S103.

The text generation system 10 repeatedly performs the above-described operations in steps S103 and S104 for a plurality of times, thus receiving a plurality of results of inferences performed the language model 22 for a single piece of input information.

A configuration of the language model 22 in the present embodiment will now be described. The language model 22 in the present embodiment is a probabilistic language model (a model that probabilistically predicts the next word to follow the preceding text and constructs sentences through this repetition). In selecting candidate words, the language model 22 randomly make a selection from among the words with high probabilities. Thus, even if the text information acquisition unit 53 repeatedly performs inference with the same information input to the language model 22, the text information received from the language model 22 is not consistent.

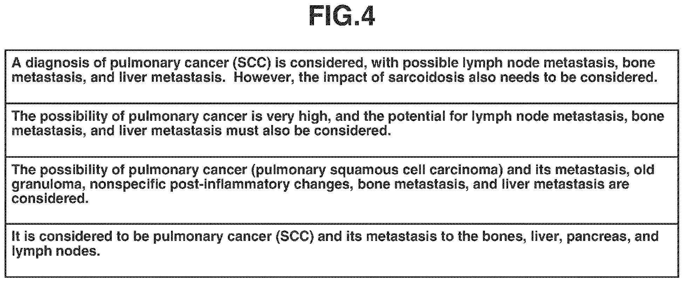

Examples of the text information that the text information acquisition unit 53 has received from the language model 22 are illustrated in Fig. 4. Fig. 4 illustrates the inference results as sentences to be described in the diagnosis section of a reading report, with each cell in the table representing the respective results each corresponding to a different one of inferences performed for a plurality of times. The inference results are different from one another, not only in expressing similar information with different strings but also in the information itself included in each inference result. For example, "SCC" and "pulmonary squamous cell carcinoma" have different expressions but the same meaning (information). In contrast, the terms "sarcoidosis" and an indication of metastasis to the spleen are not included in other pieces of text information. In this way, the text information acquisition unit 53 acquires a plurality of pieces of text information with differences arising from the randomness of the language model 22.

### (Step S105: Integrate plurality of pieces of text information)

In step S105, the determination unit 54 integrates the plurality of pieces of text information acquired by the text information acquisition unit 53 and generates a candidate for report text. The language model 22 according to the present embodiment probabilistically generates text, so that the pieces of text information are different from one another. The determination unit 54 determines that information included with high frequency in the plurality of pieces of information is important or has a high degree of certainty, and determines that information included with low frequency is not important or has a low degree of certainty (i.e., noise) that has appeared due to randomness. Examples of a possible specific method for performing this determination include a procedure where, using a medical dictionary to standardize terminology variations, information indicating possibilities (e.g., "...is considered" and "...possibility of... is/are considered...") and information indicating certainty (e.g., 'it is certain that...') are separately aggregated.

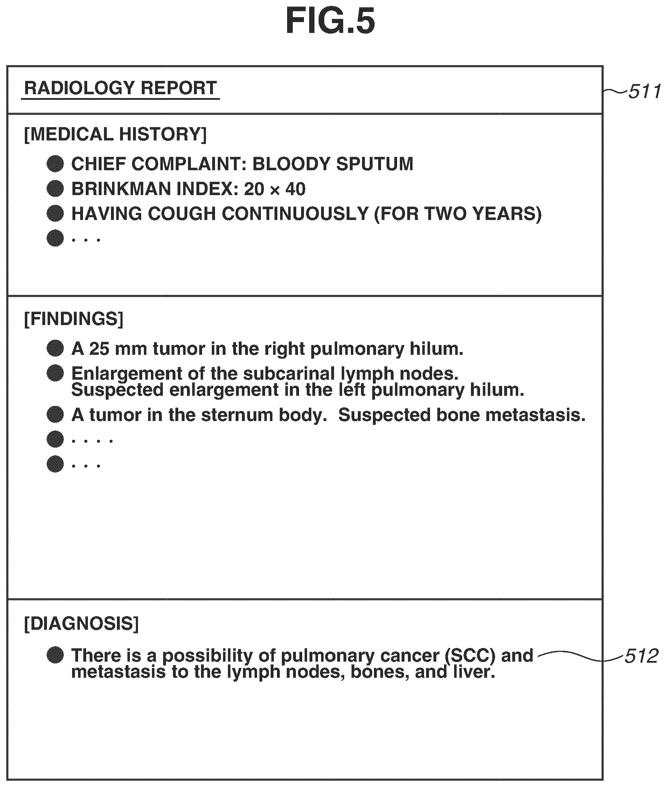

In Fig. 4, "pulmonary cancer (SCC)" appears four times, and "metastasis (lymph nodes, bone, liver)" appears more than three times as information indicating possibilities. Further, "old granuloma" and " nonspecific post-inflammatory changes" also appear one time, and their frequency is low. In contrast, no information indicating certainty is included. Thus, the determination unit 54 generates a report text candidate by combining two pieces of information with high frequency among pieces of information indicating possibilities. Fig. 5 illustrates an example where a report text candidate generated by the determination unit 54 is described in the diagnosis section 512.

The determination processing is not limited to the above-described example. For example, the text generation system 10 may instructs the language model 22 to integrate a plurality of pieces of text information, thus causing the language model 22 to generate a report text candidate. In such cases, it is desirable to prioritize information indicating certainty over information indicating possibilities, and to instruct the integration of a plurality of pieces of text information with prioritization on highfrequency information.

### (Step S106: User check and correction)

In step S106, the display control unit 55 displays the report text candidate generated by the determination unit 54 on the display unit 36 as input candidates for the radiology report. Fig. 5 illustrates an example of a display screen to be displayed on the display unit 36 by the display control unit 55. As illustrated in Fig. 5, a report text candidate generated by the determination unit 54 is displayed in the diagnosis section 512 in the radiology report 511 together with the medical history and/or findings illustrated in Fig. 3. The editing reception unit 56, after the user reviews the results, receives the information for the correction made on the diagnosis section via the operation unit 35 as appropriate, and finalizes the details of description in the reading report.

Descriptions have been provided of a case where input candidate information is text in the above examples, similar processes can be performed even if image information is combined with text.

According to the present embodiment, a plurality of inferences is performed using the language model 22 and the inference results are integrated, thus providing the user with increased stability in report text candidates even if initial inference results are unstable.

While a description has been provided of a case where the determination unit 54 generates report text candidates with prioritization of information that appears a plurality of times in step S105. Alternatively, other rules may be applied. For example, even keywords that are crucial for determining cancer metastasis should be adopted in the report text candidates, even if their frequency of occurrence is low. Such important medical information should be actively adopted in the report text candidates. The target keywords may be flagged in the medical dictionary described above, or individual keyword lists can be prepared for each reading purpose and switched according to the objective. Additionally, even if the frequency of occurrence is low due to the difficulty to infer, the information that is not included in the question text should be actively adopted in report text candidates as important findings to be emphasized. Actively adopting also low-frequency information in report text candidates in this way can provide the user with report text with a minimize possibility of oversight as well as reduced inference noise.

The above description of the present embodiment has been provided, but the present invention is not limited to these, and modifications and variations can be made within the scope of the claims.

### (Variation Example 1-1)

In the first embodiment, the display control unit 55 displays a report text candidate generated by the determination unit 54 on the display unit 36 in step S106. While this method ultimately present a report text candidate with a high degree of certainty, the non-selected information, which is the information not adopted by the determination unit 54, is hidden and cannot be utilized by the user. In the present variation example, a process is exemplified in which pieces of text information before integration together with a report text candidates generated by the determination unit 54 are presented to the user to present a report text candidate with a high degree of certainty. Additionally a process for supporting the user's correction operation is also exemplified. In other words, the display control unit 55 displays the presence or absence of non-adopted information, which has not been selected as input candidates for the report from the text information by the determination unit 54, in association with the report text candidate.

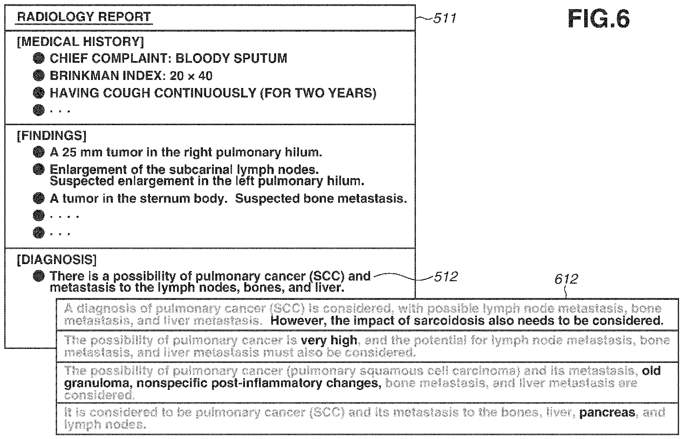

Fig. 6 illustrates a display example of a report text candidate that the text generation system 10 displays on the display unit 36 in the present variation example. Unlike Fig. 5, in Fig. 6, the display control unit 55 causes the display unit 36 to display a list of text information 612 before integration. Additionally, the characters in the range adopted in an integrated report text candidate are grayed out, distinguishing them from the information that has not been adopted (non-adopted information). In other words, the display control unit 56 displays the information adopted as input candidates for the report by the determination unit 54 and the non-adopted information in a distinguishable manner. Displaying the forms of the adopted and non-adopted information differently in this way, the user can easily identify the keywords that have not been adopted and easily understand the context of these keywords by reading the full sentences of the text information.

The user can complete a radiology report by editing a report text candidate via the operation unit 35 with reference to the pieces of text information 612 additionally displayed as illustrated in Fig. 6. The determination unit 54 may monitor the state of the user's selection operation via the operation unit 35, and may update the report text by adding the non-adopted information to the adopted information when the editing reception unit 56 detects that the user has clicked the non-adopted information included in the text information. In this way, the user can modify the report text candidate by simply clicking a non-adopted keyword included in the text information. In other words, the editing reception unit 56 receives information about editing performed by the user on the non-adopted information, and updates the report text candidate by adopting the keyword selected from the non-adopted information as the adopted information.

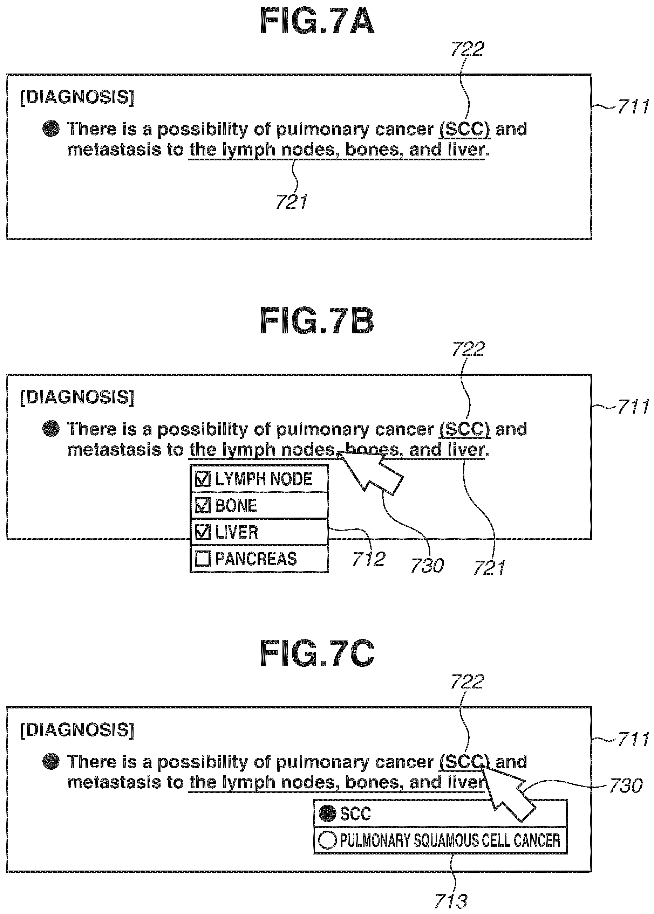

Figs. 7A to 7C illustrate an example where the pre-integrated information is utilized by selection rather than displaying the full pre-integrated information. The editing reception unit 56 displays a report text candidate in a format that allows the user to select parts of the information that are highly relevant but have not been adopted as a report text candidate due to low frequency of occurrence, rather than displaying the report sentence candidates as they are. Alternatively, the editing reception unit 56 displays, in a user selectable format, the parts that have been adopted as information but not adopted in the integration process. In Fig. 7A, to indicate the presence of a part of the metastasis sites (pancreas) that has not been adopted as a report text candidate, the parts of a sentence adopted as a report text candidate are underlined with an underline 721 to indicate that the parts are selectable. Additionally, to indicate the presence of unified information (SCC, pulmonary squamous cell carcinoma) to which variations in expression in the pre-integrated information have been unified, the unified parts (SCC) are underlined with and underline 722 to indicate they are selectable. In Fig. 7B, information 712 in the form of checkboxes including non-adopted information is displayed. When the user moves the mouse pointer 730 over the underline 721 indicating the list of metastasis sites, the editing reception unit 56 displays the adopted and non-adopted information as the information 712 in the form of checkboxes. Lymph nodes, bones, and liver are already adopted and thus checked in the corresponding checkbox, while the pancreas is not adopted and thus unchecked in the corresponding checkbox. The user can correct the metastasis sites by toggling the checkboxes on or off. In Fig. 7C, pre-integrated terms are displayed as information 713 in the form of radio buttons. When the user moves the mouse pointer 730 over the information 722 indicating the list of metastasis sites, the editing reception unit 56 displays the list of pre-integrated terms as the information 713 in the form of radio buttons. Since SCC is adopted among SCC and pulmonary squamous cell carcinoma, SCC is in a selected state. The user can modify terminology by selecting the corresponding term with the radio buttons.

The display control unit 55 may display parts of the report sentence candidates that include non-adopted information in an easily visually distinguishable format, such as underlining, coloring the text or background, or using bold text, on the display unit 36. Additionally, the display control unit 55 may initially display nothing altered, and only display available options when a selection is made by a mouse or when the target range enters an editable state.

According to the present variation example, pre-integrated information is presented to the user, thus producing effect of providing additional supplementary information for the correction of the report sentences.

### (Variation Example 1-2)

In the above-described embodiment and the variation example, a report text candidate is edited by the user, as an example. This method is advantageous in that the final report text can be created just as intended by the user. However, depending on a case, similar correction may be to be frequently performed. For example, the user may frequently need to correct the expression of words (using different words with the same meaning) and the tone or style of sentences in the report text candidate obtained for the report text. In the present variation example, the editing reception unit 56 reflects the details of user's editing on a report text candidate in the next report text generation to be performed by the determination unit 54. In this way, a function for correcting the report text candidate is provided to the user, and the user is also prevented from repeatedly performing the similar correction.

When the user modifies the report text candidate in the radiology report illustrated in Fig. 5 or 7, the editing reception unit 56 transmits the editing details to the determination unit 54. The determination unit 54 sets the received editing details as a condition to be applied when integration is performed next time, and uses it when pieces of text information are integrated in step S105 next time.

For example, in a case where the user changes "SCC" to " pulmonary squamous cell carcinoma" in Fig. 7C, the editing reception unit 56 notifies the determination unit 54 of the change. Based on the received information, the determination unit 54 lowers a selection priority level of the term "SCC", and rewrites and raises a selection priority level of the term "pulmonary squamous cell carcinoma" in the dictionary used for integration. In this way, "pulmonary squamous cell carcinoma" can preferentially be adopted as a report text candidate instead of "SCC" in a case where a term meaning "SCC" or "pulmonary squamous cell carcinoma" is output when the report text candidate is generated next time.

Additionally, for example, if the user corrects the tone of the report text candidate from "Possibility exists..." to "There is a possibility...", the editing reception unit 56 similarly notifies the determination unit 54 that the tone has been changed to "polite form". Based on the received information, the determination unit 54 changes a tone parameter from "plain form" to "polite form", thereby changing the tone to "formal style" in connecting the selected information in step S105 to be performed next.

According to the present variation example, reflecting the details of the user's edits in the determination unit 54 makes it possible to provide a mechanism that automatically brings the form of the report text candidates for the subsequent reports closer to the user's desired style based on the correction results for the previous report text. This reduces the burden of editing work for the user.

In the above examples, a selection priority level of a term used for the report text candidate and a parameter related to a tone of words for the report text candidate are changed. However, the implementation of the present invention is not limited to this. For example, report text edited by the user may be recorded, and the determination unit 54 sets a condition in the next processing. For example, when the determination unit 54 requests the language model 22 to integrate pieces of text information, the determination unit 54 may set a condition, such as "Generate report sentences similar to the following report sentences: ...(the previously recorded user-edited report sentence is described here) ...". Additionally, a plurality of pieces of text information and user-edited report text may be passed to the determination unit 54. In requesting the language model 22 to integrate text information, the determination unit 54 may set a condition such as "Below are samples of the report sentence before and after integration. Integrate text information this time in a manner similar to these samples: ... (hereafter, the text information and report sentences are described) ...". This enables the generation of text that is closer to the user's desired style, thereby reducing the burden of editing work for the user.

### (Variation Example 1-3)

In the above-described embodiment and the variation examples, a plurality of pieces of text information are acquired through a plurality of times of inferences by inputting the same input information to the one language model 22 for a plurality of times. This method can present the user with a report text candidate that is less influenced by the randomness of the language model 22. However, there is a possibility that a report text candidate biased toward a specific language model is generated. In the present variation example, an example process will be illustrated in which the text information acquisition unit 53 performs inference by using a plurality of language models different from one another to generate report text candidates not biased toward a specific language model.

The text information acquisition unit 53 performs inference by inputting the input information generated by the generation unit 52 to a plurality of language models 22 in step S103, and acquires the results from the language models 22 in step S104. At this time, each of the language models 22 may perform inference one time, or may perform inference more than one time. The determination unit 54 generates a report text candidate by integrating pieces of text information that the text information acquisition unit 53 has acquired from the plurality of language models 22.

According to the present variation example, the determination unit 54 can acquire a report text candidate that is not biased to a specific language model by integrating the inference results acquired from the plurality of language models.

### (Variation Example 1-4)

In the above-described first embodiment, the generation unit 52 converts acquired input candidate information into input information. However, the implementation of the present invention is not limited to this. For example, the input candidate information acquisition unit 51 may omit the operation in step S102 described in the first embodiment, and may directly input the input candidate information acquired through the user input to the language model 22 as the input information.

For example, a model specialized in generating report text from free text may be used as the language model 22, and the input candidate information illustrated in the first embodiment may be directly inferred.

This configuration eliminates the need to add inference conditions to the input candidate information, allowing the language model 22 to directly generate text information from the input candidate information.

The text information acquisition unit 53 may input the input candidate information acquired through the user's input together with previously stored inference request template sentences to the language model 22, to omit the operation in step S102. Examples of the template sentences include "Generate report sentences similar to the attached report sentences". This configuration eliminates the need to add inference conditions to the input candidate information, allowing the language model 22 to directly generate text information without processing the input candidate information.

A second embodiment of the present invention will now be described. In the above-described first embodiment, a plurality of times of inferences is performed by the language model 22 based on the same input information, and results of the inferences are integrated. This configuration controls the randomness of the language model and biases between language models, thereby providing an example of stably generating report text candidates that include important information.

In the second embodiment, the generation unit 52 provides a plurality of pieces of input information different from one another to the at least one of language model 22, causes the language model 22 to perform a plurality of times of inferences based on the pieces of input information, and integrates the results. This reduces the instability in generating report text candidates caused by the instability of the input candidate information provided by the user. According to the present invention, it is possible to provide the user with robust report text candidates against the instability of the input candidate information entered by the user.

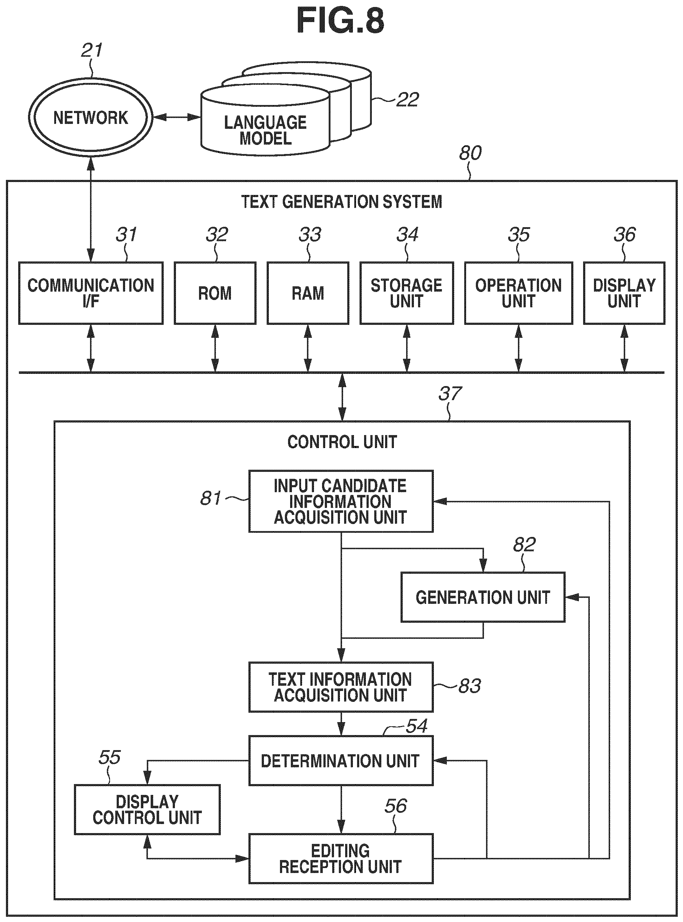

The configuration of the present embodiment will be described with reference to Fig. 8, focusing only on the parts that differ from the first embodiment in relation to the text generation system 80. An input candidate information acquisition unit 81 acquires the information to be processed (input candidate information) from the operation unit 35. This information includes parameters entered by a doctor or radiogram interpretation doctor. In other words, the input candidate information acquisition unit 81 corresponds to an example of an input candidate acquisition unit configured to acquire input candidate information. In this embodiment, an example is described in which parameters such as the primary site and primary size used for report generation are acquired as input candidate information, but other parameters may also be acquired as input candidate information.

As in the first embodiment, a generation unit 82 generates input information from the input candidate information acquired from the input candidate information acquisition unit 81. The generation unit 82 further generates another piece of input candidate information with the parameter of the input candidate information partially changed, and generates new input information from the other piece of input candidate information.

A text information acquisition unit 83 causes the language model 22 to perform inference on the individual pieces of input information generated by the generation unit 82, thus acquiring a plurality of pieces of text information.

The determination unit 54, the display control unit 55, and the editing reception unit 56 perform the operations similar to those described in the first embodiment.

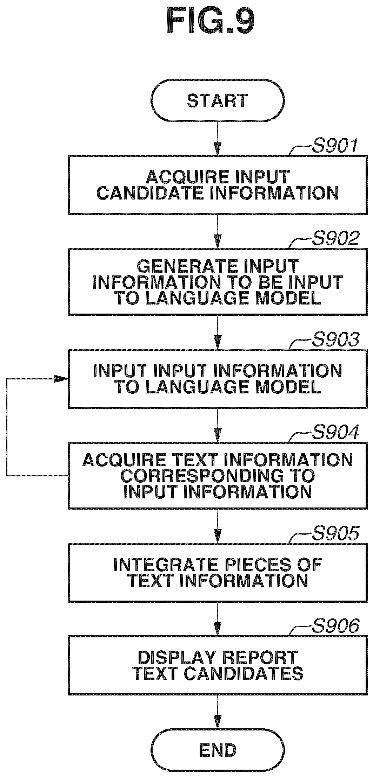

Next, processing procedures performed by the text generation system 80 according to the present embodiment are described. The processing procedures performed by the text generation system 80 according to the present embodiment are described with reference to Fig. 9.

In the present embodiment, a description will be provided of an example in which report sentences for a radiology report about the primary pulmonary cancer are generated from the parameters specified by the user. The present embodiment is also applicable to generation of report sentences other than the pulmonary cancer and generation of text other than the radiology report.

### (Step S901: Acquire input candidate information)

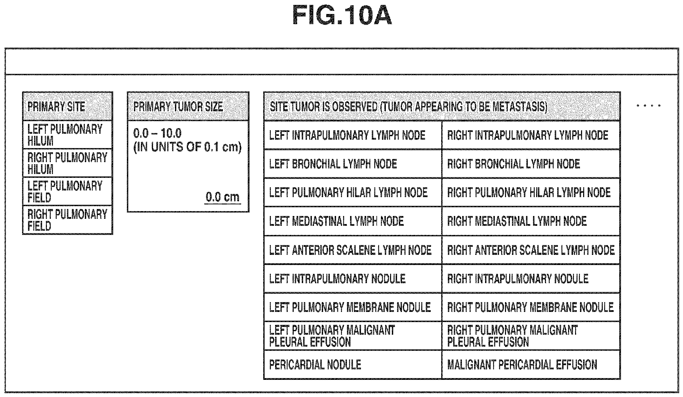

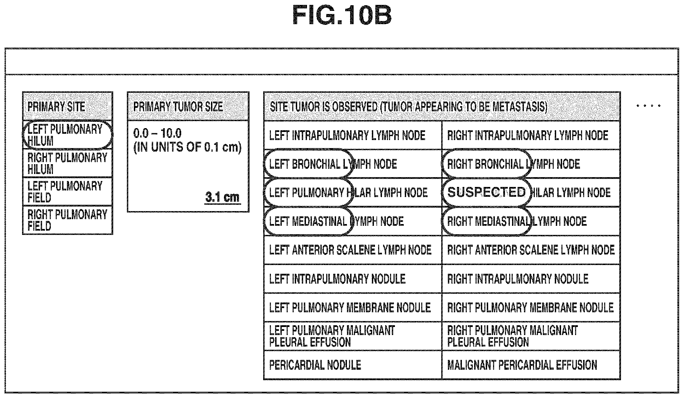

In step S901, the input candidate information acquisition unit 81 acquires parameters input by the user via the operation unit 35, and stores the parameters in the RAM 33. Examples of the parameters input by the user are illustrated in Figs. 10A and 10B. Fig. 10A illustrates a dialogue (i.e., an example of an input template) to which the user inputs parameters. The dialogue presents options for determining metastasis status for primary pulmonary cancer. The display control unit 55 controls the display unit 36 to display the dialogue. The text generation system 80 acquires information input to the dialogue by the user via the operation unit 35. In order to simplify the descriptions, in the present embodiment, parameters for a primary site, a primary tumor size, and a site of metastasis are allowed to be input. In practice, other parameters to be used for the diagnosis may also be input, or parameters which can be input may be narrowed down in order to avoid complication of the dialog. Fig. 10B illustrates examples of the parameters that the user has actually input. In the present embodiment, the user has determined that the primary site is the left pulmonary hilum, and is allowed to specify a portion describing "Left Pulmonary Hilum" in the dialogue by operating the operation unit 35. The display control unit 55 displays a mark on the specified portion to enable the dialogue to be visually recognizable. The user has determined that a primary tumor size is "3.1 cm". The display control unit 55 similarly acquires a determined size input by the user through the operation performed on the operation unit 35, and displays the result as a numerical value on the dialogue. Further, the user points out "Right Pulmonary Hilar Lymph Node" as a site having high probability of metastasis, and points out a plurality of sites as sites having low probability of metastases. The user can similarly specify these sites by operating the operation unit 35. In the present embodiment, the input candidate information acquisition unit 81 acquires the above-described radiology reports as the input candidate information.

### (Step S902: Input Information Generation)

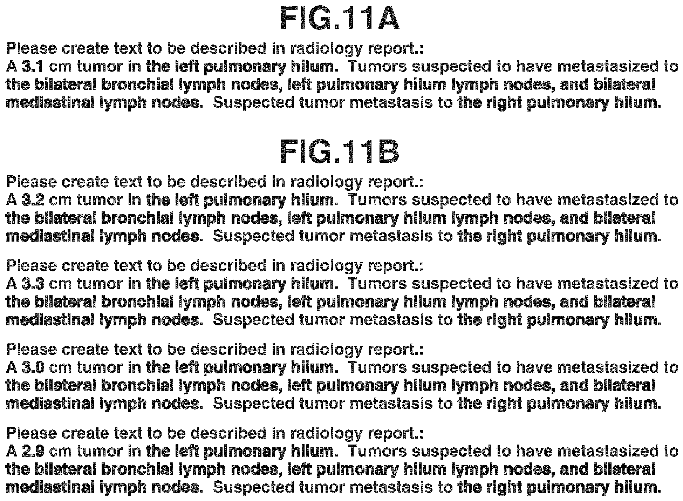

In step S902, the generation unit 82 generates a plurality of pieces of input information that can be inferred by the language model 22 using parameters acquired by the input candidate information acquisition unit 81. Examples of the input information generated by the generation unit 82 are illustrated in Figs. 11A and 11B. Fig. 11A illustrates input information generated by directly converting the input candidate information acquired in step S901 into text. As an inference condition to be set when the language model 22 performs inference in step S904 described below, a sentence "Please create text to be described in radiology report." is added to the input information. Here, information about a primary tumor, " A 3.1 cm tumor in the left pulmonary hilum.", and information about sites of metastases, "the bilateral bronchial lymph nodes, left pulmonary hilum lymph nodes, and bilateral mediastinal lymph nodes" can be generated through a known technique, such as inserting the input candidate information acquired in step S101 into template text.

The generation unit 82 further generates input information different from the input information in Fig. 11A by partially changing the input candidate information. In other words, the generation unit 82 generates first input information including input candidate information selected by the user for the input template, and second input information including other pieces of input candidate information selected by the user. Fig. 11B illustrates a plurality of pieces of input information generated by the generation unit 82 using the tumor size of 3.1 cm obtained as input candidate information, adjusted in increments of 0.1 cm. In this way, the generation unit 82 generates plurality of pieces of different input information from the acquired parameters. The reason for slightly varying the input candidate information provided by the user is based on the implicit assumption that there is instability or ambiguity in the input candidate information provided by the user. In other words, the tumor size of "3.1 cm" entered by the user in the present embodiment may vary slightly when entered by a different user due to differences in measurement methods or rounding methods below the second decimal place between users. Such slight variations can affect the inference results of the language model 22. More specifically, if the user provides a tumor size of 3.2 cm, important results that would have been obtained from the inference by the language model may not be obtained if 3.1 cm is provided instead. Therefore, in the present embodiment, the inference by the language model is performed even when slight variations are intentionally added to the input candidate information provided by the user, and the results are integrated using the method described below.

In the above description, a description has been provided of an example in which a plurality of pieces of input information with slight variations to the tumor size is generated, but the implementation of the present invention is not limited to this. For example, slight variations may be made to the location of the primary site or the metastasis site (such as changing to adjacent sites), or information about certainty of metastasis may be varied (such as changing "suspect" to "strongly suspect" or "possible").

### (Step S903: Enter input information to the language model)

In step S903, the text information acquisition unit 83 inputs each of the plurality of pieces of input information generated by the generation unit 82 to the language model 22. Thus, the language model 22 performs inference(s) for the respective pieces of input information.

### (Step S904: Acquire text information)

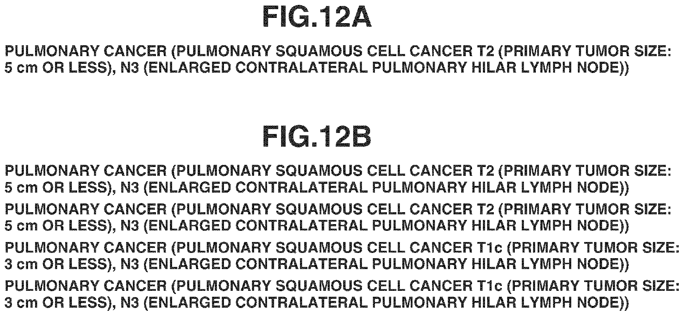

In step S904, the text information acquisition unit 83 acquires results of inferences performed by the language model 22 in step S903 described above. The language model 22 performs inference based on a plurality of pieces of different input information, pieces of text information received by the text information acquisition unit 83 is not the same in some cases. Figs. 12A and 12B illustrate examples of details of pieces of text information received by the text information acquisition unit 83 from the language model 22. To simplify the descriptions of the present embodiment, in Figs. 12A and 12B, unlike the example of text information in Fig. 4, the details of the plurality of pieces of text information obtained from the language model 22 are described in a unified form that allows for comparison. Fig. 12A illustrates the details of pieces of text information obtained through inference based on the input information illustrated in Fig. 11A, including information about the primary tumor size determination (T2/5 cm or less) and metastasis range determination (N3/ enlarged contralateral pulmonary hilar lymph node). In contrast, Fig. 12B illustrates the information obtained through inference from each piece of input information of Fig. 11B, and unlike Fig. 12A, there is a difference in the primary tumor size determination. The number of inferences for each piece of input information in step S903 can be once for each piece of input information or a plurality of times as in the first embodiment. Even if inference is performed once for each piece of input information, a plurality of times of inferences is to be performed using similar input information, which can reduce the computational cost while controlling some degree of inference instability. In addition, each piece of input information is subjected to inference for a plurality of times, so that more stable report text candidates is obtained, as in the first embodiment.

### (Steps S905 and S906: Operations Similar to the first embodiment)

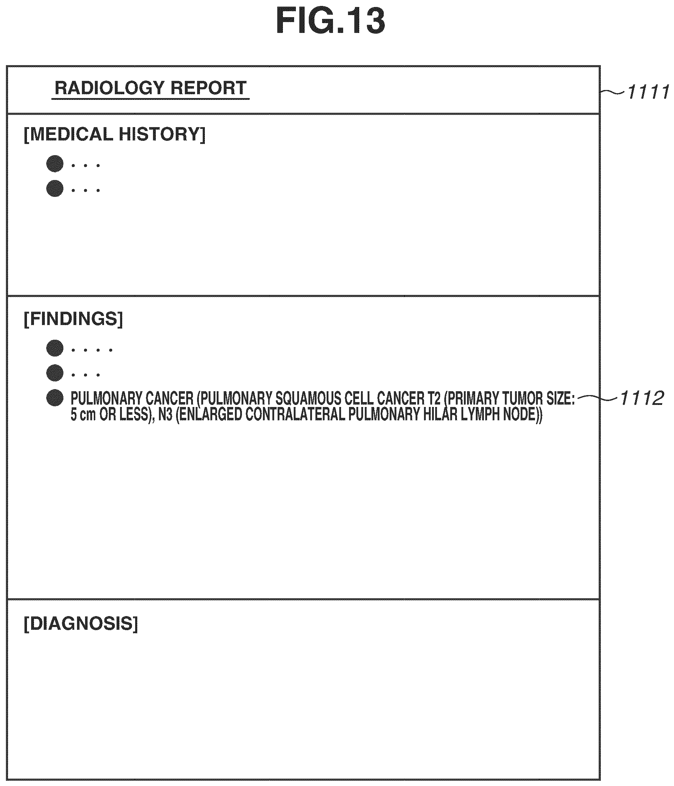

As in the first embodiment, in steps S905 and S906, the determination unit 54 integrates the pieces of text information acquired by the text information acquisition unit 53, and the editing reception unit 56 displays a report text candidate integrated by the determination unit 54 on the display unit 36. As illustrated in Figs. 12A and 12B, pieces of different information, "T2: 5 cm or less" and "T1c: 3 cm or less", are acquired. However, the former appears three times, while the latter appears twice. Therefore, by means of a majority decision, 'T2/5 cm or less' is adopted to generate the final report text. Fig. 13 illustrates an example where the final report sentence candidate 1112 is displayed on the reading report 1111.

According to the present embodiment, the text generation system 80 causes the language model 22 to perform inference based on the plurality of types of input information and the results are integrated, thus providing the user with stable report sentence candidates that are less affected by the instability and ambiguity of user input.

In addition to the method of generating input information for the language model 22 by applying slight variations to the input candidate information as exemplified above, the description of the inference conditions provided by the generation unit 82 can also be changed. Replacing the inference condition illustrated in Figs. 11A and 11B, "Please create text to be described in radiology report" with "Please create report text to be described in the findings column" generates a plurality of input sentences. This enables obtainment of a plurality of pieces of text information that varies due to the instability of the input. Thus integrating the text information obtained in this way enables the user to be provided with report sentence candidates that are less affected by the instability of the user's input.

### (Variation Example 2-1)

In the second embodiment, a plurality of pieces of input information is generated in step S902. As in the first embodiment, with this method, information not adopted by the determination unit 54 is hidden and cannot be used by the user. In this variation example, the user is presented with the pre-integrated text information along with the report text candidates generated by the determination unit 54, thus providing report text candidates with a high degree of certainty as in the variation example 1-1. In addition, the present variation example exemplifies a process for assisting the user's correction work.

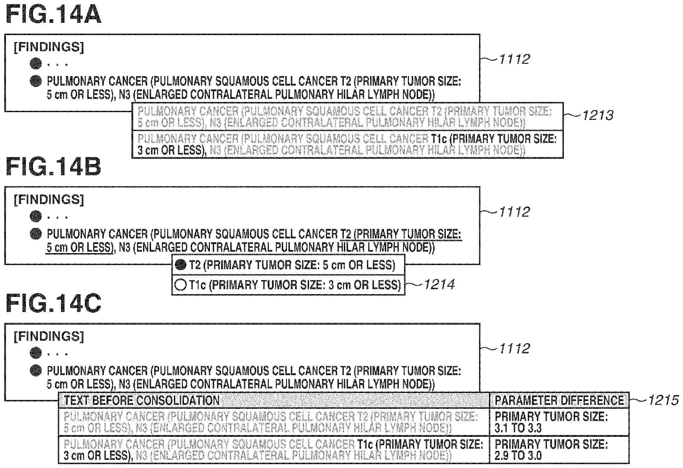

Figs. 14A to 14C illustrate display examples of report text candidates displayed on the display unit 36 by the display control unit 55 included in the text generation system 80 according to the present variation example. Unlike the display example illustrated in Fig. 13, in Fig. 14A, the display control unit 55 displays pieces of text information 1213 before integration on the findings column 1112. Additionally, the display control unit 55 grays out the characters within the range adopted in the integrated report text candidate, allowing the user to easily identify the keywords that are not included in the integration. Furthermore, as illustrated in a field 1214 of Fig. 14B, the display control unit 55 may display the characters not adopted in the report text candidate in a radio button format. Additionally, as illustrated in a field 1215 of Fig. 14C, the display control unit 55 may display the differences in parameters in obtaining each piece of text information that forms the basis of the report text candidate, in a different display format from Fig. 14A and 14B. This allows the user to easily infer the reasons why certain characters in the text information are not adopted in the report sentence text.

According to the present variation example, pieces of text information before being integrated to the report text candidate are also presented to the user, thus providing the user with additional supplementary information for the correction of the report text candidate.

### (Variation Example 2-2)

In the second embodiment and the variation examples, a method for providing a mechanism that allows the user to correct the report text candidate has been described. As described in conjunction with the variation example 1-2, this method may require the user to perform similar corrections frequently. In this variation example, a process is exemplified in which the editing reception unit 56 reflects, in the next report text generation, the details edited by the user in the report text candidate, providing a correction function for the report text candidate, as well as a process for preventing the user from having to repeat similar corrections. In the variation example 1-2, the details of user's corrections are input to the determination unit 54 for use in the next process, but in this variation example, they are input to the generation unit 82 for use in the next process.

When the user corrects the report text candidate based on the display illustrated in Fig. 13 and Figs. 14A to 14C, the editing reception unit 56 transmits the editing details to the generation unit 82. The generation unit 82 sets the received editing details as conditions to the conditions for the next and subsequent input generation, and uses the conditions in the next input information generation in step S902.

For example, in a case where the user changes a description of a primary tumor size from "T2" to "T1c" based on the display illustrated in Fig. 14B, the editing reception unit 56 notifies the generation unit 82 of information about a difference between conditions for parameters when a difference between primary tumor sizes "T2" and "T1c" occurs. Specifically, the editing reception unit 56 transmits information about a parameter "Primary Tumor Size: 3.1 cm" input by the user and a parameter "Primary Tumor Size: 2.9 cm to 3.0 cm" when the inference result indicating a primary tumor size T1c is acquired to the generation unit 82. From this information, the generation unit 52 calculates a modification value for a primary tumor size, "-0.2cm to - 0.1cm", which is a correction value to be used when input information is generated next time, and adds the inference condition "Note that a primary tumor size should be adjusted by -0.2 cm to -0.1 cm from the information described below" to the next input information. In this way, automatically applying the primary tumor size adjustment to the input information for subsequent instances enables text information with the adjusted primary tumor size to be obtained from the language model 22.

According to the present variation example, the user's editing details are reflected on the generation unit 82, so that the report text candidates for the subsequent instances are automatically brought closer to the corrections made to the previous report sentence. This brings the report text candidates for the subsequent instances closer to the user's expectations, thereby reducing the user's editing workload.

The user's correcting details may be input to the input candidate information acquisition unit 81 instead of the generation unit 82. As illustrated in Fig. 10A, pieces of information which can be displayed on a screen are limited. Therefore, it is not possible to display selection items which cover all of the possibilities. Thus, the input candidate information acquisition unit 81 adds selection items to be used to reproduce the user's correction, or deletes selection items unnecessary for reproduction of the correction. Then, the input candidate information acquisition unit 81 can dynamically change the dialogue to be displayed to the user in step S901 next time. Specifically, if the user adds descriptions related to abdominal organs as information indicating a site of a metastasis to the report sentence, the input candidate information acquisition unit 81 adds items of various abdominal organs to the list of tumor confirmation sites from the next instance. If the user adds descriptions of various blood test indicators to the report sentence, the input candidate information acquisition unit 81 adds a list of blood test result options from the next instance. If the user deletes information about the primary tumor size from the report text, the input candidate information acquisition unit 81 removes the input field for the primary tumor size from the next instance. These methods can be considered. Thus, the report sentence candidates for the next and subsequent instances are brought closer to the user's expectations, thereby reducing the user's editing workload.

In the above-described embodiments, information regarding the parameters used for generating input information in the input candidate information acquisition unit 81 has been described as an example, but the implementation of the present invention is not limited to this. For instance, the report text edited by the user can be recorded, and in the next process, the generation unit 82 can add a description such as "Generate a report sentence similar to the following report sentence: ... (hereafter, report sentence follows)..." in generating input information. This easily establishes a mechanism that enables the user to avoid repeating similar corrections.

### (Variation Example 2-3)

In the above-described second embodiment, the generation unit 82 acquires parameters selected by the user from the options displayed on the dialogue, and generates a plurality of pieces of input information by adding variations to the parameters. However, the implementation of the present invention is not limited to this. For example, as illustrated in the first embodiment, the generation unit 82 may analyze the input candidate information which is free text, to extract keywords and numerical values which are to be served as parameters, and then apply variations to the parameters in the corresponding portions. By automatically detecting parameters and applying variations to the parameters in this way, there is no need for the user to create a dialog to select parameters, and the user can freely input information without having to be aware of how to use the dialog.

Additionally, for items that remain ambiguous during automatic analysis, the range of parameter variation can be increased. Adjusting the range of variation in this way produces a beneficial effect of generating input information that reflects the instability and ambiguity of the user's input.

The above is an example of embodiments, but the present invention is not limited to the embodiments described above and in the accompanying drawings. It can be appropriately modified and implemented within the scope that does not change the gist of the invention.

### <Other Embodiments>

A technique according to the present disclosure can take various forms such as a system, an apparatus, a method, a program, and a recording medium (storage medium). Specifically, the technique according to the present disclosure may be applied to a system consisting of a plurality of devices (such as a host computer, an interface device, an image capturing apparatus, and a Web application), or an apparatus consisting of one device.

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc^{™} (BD)), a flash memory device, a memory card, and the like.

## Claims

1. A text generation system, comprising:
an input candidate information acquisition means for acquiring input candidate information to be input to a language model configured to output text information from input information;
a text information acquisition means for acquiring a plurality of pieces of text information different from one another by inputting input information based on the input candidate information to the language model; and
a determination means for determining a text candidate by using the plurality of pieces of text information.

2. The text generation system according to claim 1, further comprising a generation means for generating the input information to be input to the language model based on the input candidate information.

3. The text generation system according to claim 2,
wherein the generation means generates a plurality of pieces of input information different from one another based on the input candidate information, and
wherein the text information acquisition means acquires the plurality of pieces of text information by inputting the plurality of pieces of input information to the language model.

4. The text generation system according to claim 3,
wherein the input candidate information acquisition means acquires a selection that a user has made for input template as input candidate information, and
wherein the generation means generates first input information including the input candidate information selected by the user and second input information including another piece of input candidate information related to the input candidate information selected by the user.

5. The text generation system according to any one of claim 1 to 3, wherein the determination means determines the text candidate by further inputting the plurality of pieces of text information to the language model.

6. The text generation system according to any one of claim 1 to 3, further comprising:
a display control means for displaying the text candidate determined by the determination means on a display means; and
an editing reception means for receiving information about editing that is to be reflected on a report and is performed by a user on the text candidate.

7. The text generation system according to claim 6, wherein the display control means displays adopted information adopted by the determination means as an input candidate to be input to a report from the text information and non-adopted information not adopted by the determination means in a distinguishable manner.

8. The text generation system according to claim 6, wherein the display control means displays the presence or absence of non-adopted information not adopted by the determination means as an input candidate to be input to the report from the text information, in association with the text candidate.

9. The text generation system according to claim 7 or 8, wherein the editing reception means receives information about editing performed by the user on the non-adopted information, and updates the text candidate with a keyword selected from the non-adopted information serving as adopted information.

10. The text generation system according to claim 2,
wherein the generation means updates a rule for generating input information based on the information about editing performed by the user, received by the editing reception means, and generates the input information based on the updated rule.

11. The text generation system according to claim 6, wherein the determination means updates a rule for determining the text candidate based on the information about editing performed by the user, received by the editing reception means, and determines the text candidate based on the updated rule.

12. The text generation system according to claim 11, wherein the determination means changes a priority level of a keyword to be adopted as a text candidate based on the information about editing performed by the user.

13. The text generation system according to any one of claim 1 to 3, wherein the language model includes a plurality of language models different from one another, and
wherein the acquisition means acquires the plurality of pieces of text information by inputting input information to the plurality of language models.

14. A text generation method, comprising:
acquiring input candidate information to be input to a language model configured to output text information from input information;
acquiring a plurality of pieces of text information different from one another by inputting input information based on the input candidate information to the language model; and
determining a text candidate by using the plurality of pieces of text information.

15. A storage medium storing a program for causing a computer to execute the text generation method according to claim 14.
